(19) Europäisches Patentamt
European Patent Office
Office européen des brevets

(11) **EP 4 619 065 B1**

(12) **EUROPEAN PATENT SPECIFICATION**

(45) Date of publication and mention
of the grant of the patent:
**03.06.2026 Bulletin 2026/23**

(21) Application number: **23802193.5**

(22) Date of filing: **07.11.2023**

(51) International Patent Classification (IPC):
***A61M 16/00*** *(2006.01)*

(52) Cooperative Patent Classification (CPC):
**A61M 16/024;** A61M 2016/0027; A61M 2230/46;
A61M 2230/60

(86) International application number:
**PCT/EP2023/080898**

(87) International publication number:
**WO 2024/104812 (23.05.2024 Gazette 2024/21)**

(54) **SYSTEMS FOR ULTRASOUND-BASED ASSESSMENT OF REGIONAL RESPIRATORY PARAMETERS TO GUIDE LUNG PROTECTIVE VENTILATION**

SYSTEME ZUR ULTRASCHALLBASIERTEN BEURTEILUNG REGIONALER ATEMPARAMETER ZUR FÜHRUNG VON LUNGENSCHUTZVENTILATION

SYSTÈMES D'ÉVALUATION PAR ÉCHOGRAPHIE DE PARAMÈTRES RESPIRATOIRES RÉGIONAUX À DES FINS DE GUIDAGE D'UNE VENTILATION DE PROTECTION PULMONAIRE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **17.11.2022 US 202263426069 P**

(43) Date of publication of application:
**24.09.2025 Bulletin 2025/39**

(73) Proprietor: **Koninklijke Philips N.V.**
**5656 AE Eindhoven (NL)**

(72) Inventors:
• **HAARTSEN, Jaap Roger**
**5656 AG Eindhoven (NL)**
• **VISSER, Jeffrey**
**5656 AG Eindhoven (NL)**
• **BUIZZA, Roberto**
**5656 AG Eindhoven (NL)**
• **HENDRIKS, Cornelis Petrus**
**5656 AG Eindhoven (NL)**
• **WIEMKER, Rafael**
**5656AG Eindhoven (NL)**
• **KOEHLER, Thomas**
**5656AG Eindhoven (NL)**
• **WIEBERNEIT, Nataly**
**5656AG Eindhoven (NL)**
• **SABCZYNSKI, Joerg**
**5656AG Eindhoven (NL)**
• **POLKEY, Michael**
**5656AG Eindhoven (NL)**

(74) Representative: **Philips Intellectual Property & Standards**
**High Tech Campus 52**
**5656 AG Eindhoven (NL)**

(56) References cited:
EP-B1- 1 292 224      US-A1- 2013 025 596
US-A1- 2014 373 845      US-A1- 2015 290 418
US-A1- 2020 121 877

**Description**

**CROSS-REFERENCE TO RELATED APPLICATIONS**

This patent application claims the priority benefit under 35 U.S.C. § 119(e) of U.S. Provisional Application No. 63/426,069, filed on November 17, 2022.

The following relates generally to the respiratory therapy arts, respiratory stress and strain arts, Ventilator Induced Lung Injury (VILI) risk assessment arts, and related arts.

**BACKGROUND**

Assessment of VILI risk in mechanically ventilated patients is commonly done based on information such as the ventilator settings and measured pressure and airflow. However, these provide global information on the operation of the lungs as a whole. Patients with lung abnormalities such as consolidations, atelectasis, chronic obstructive pulmonary disease (COPD), or acute respiratory distress syndrome (ARDS) often show regional variations in lung resistance and compliance. The affected parts of the lung with a low compliance may show tidal recruitment (i.e., a repetitive opening and closing of collapsed alveoli during the mechanical respiratory cycle). The resultant high tissue stress in these atelectatic zones can trigger a local inflammatory response and injure the alveolar-capillary membrane called atelectotrauma which potentially can lead to ventilator-induced lung injury (VILI), impairing the outcome of these patients. Measurement of global lung parameters may fail to detect these more localized regional stresses. When mechanically ventilated, these patients may benefit from lung recruitment maneuvers and higher Positive End-Expiratory Pressure (PEEP) levels to avoid atelectotrauma by maintaining consistent lung opening of the parts of the lung with a low compliance. However, this may result in regional overdistension in those (healthy) parts of the lung having a high compliance, which is another mechanism of VILI, impairing the outcome of patients.

For these patients, the use of advanced imaging techniques can help to determine a lung protective ventilation strategy. Currently, computed tomography (CT) scanning is preferred to obtain reliable regional information by measuring the difference in aeration observed between static images taken at end-inspiration and end-expiration. However, this method is time consuming, exposes the patient to radiation, and cannot be applied at the bedside. Another technique to obtain regional lung information is Electrical Impedance Tomography (EIT). However, also this technique is expensive and not widely accessible.

Another solution to obtain regional lung information is the use of lung ultrasound (LUS), a subclass of diagnostic ultrasound. For example, a BLUE protocol (see, e.g., Lichtenstein DA et al. Relevance of lung ultrasound in the diagnosis of acute respiratory failure: the BLUE protocol. Chest. 2008; Lichtenstein, D.A. Lung ultrasound in the critically ill. Ann. Intensive Care 4, 2014) allows for diagnosis of acute respiratory failure at the bedside by assessing and scoring for each lung zone a multitude of 'signs' in the ultrasound image. Each sign or a combination of signs is associated with a lung disorder (e.g., the presence of B-lines in combination with lung rockets indicating interstitial syndrome). Also, LUS can be used to assess tidal recruitment Tusman G, et al. Real-time images of tidal recruitment using lung ultrasound. Crit Ultrasound J. 2015).

An interesting lung phenomenon that can be visualized by LUS is lung sliding, which is the sliding back and forth of the visceral and parietal pleura relative to one another as the patient breathes. Lung sliding can be assessed qualitatively by ultrasound at the anterior, lateral, and posterior chest and is mainly used for the diagnosis of pneumothorax. Recent advances in ultrasound image processing such as speckle tracking allow for quantification of lung sliding (see, e.g., G. Duclos et al. "Speckle tracking quantification of lung sliding for the diagnosis of pneumothorax: A multicentric observational study", Intensive Care Med. 2019; G. Duclos et al. "A picture's worth a thousand words: Speckle tracking for quantification and assessment of lung sliding", Intensive care med. 2019; E. Fissore et al. "Pneumothorax diagnosis with lung sliding quantification by speckle tracking: A prospective multicentric observational study", The American journal of emergency medicine 2021; and L. Crognier et al. "Diaphragmatic speckle tracking imaging for 2D-strain assessment in mechanical ventilation weaning test", Medical hypotheses 2021). These speckles are acoustic markers which are formed by interference of ultrasound waves that are scattered from physical structures of a size comparable to the wavelength of the ultrasound waves. In some examples, a measure for lung sliding is obtained by calculating the average temporal intensity changes of the ultrasound speckles in the pleural line. This results in an Intensity Based Speckle Tracking (IBST) score waveform which correlates well with the ventilator flow waveform.

Another method to obtain information on presence of tidal recruitment and overdistension is to analyse the shape of the pressure-time curve. For constant flow and in the absence of respiratory muscle activity, the status of the lung can be assessed by defining a stress index based on the concavity of the pressure-time curve. However, this can only be done on a global basis and not for specific parts of the lung.

Yet another method to obtain information on presence of atelectasis and overdistension is to analyse the shape of the pressure-volume (P-V) loop, using the pressure at the wye or using transpulmonary pressure e.g., derived from

**EP 4 619 065 B1**

esophageal pressure measurements. Here, depending on the shape of the P-V loop zones of overdistension as one option, and decruitment and atelectasis as the other option can be distinguished. Again, only global information on the lungs is thusly obtained. The method based on the transpulmonary pressure is more accurate but not always available at the bedside.

Another method to guide a lung protective ventilation strategy is based on assessment of lung resistance and compliance. Methods to assess global lung resistance and compliance exist. These methods are based on dedicated ventilator maneuvers to obtain the parameters needed to solve an element model for lung resistance and compliance. However, since it is difficult to estimate the respiratory drive, these methods only work for a passive patient, i.e., Pmus = 0 cm $H_2O$. Moreover, these methods only work for global resistance and compliance and cannot give a good approximation of regional variations in lung resistance and compliance, and hence of regional tidal recruitment and overdistension.

US patent application n°2015290418A1 also discloses a system and method for targeted delivery of agent for treatment of a respiratory condition. A respiratory characteristic is measured, and an agent is delivered based on the respiratory characteristic during a respiratory cycle. A computer-controlled system is used to control mixing of an agent into the fluid delivery channel of a ventilator to treat the respiratory condition of a patient. Various lung measurements for targeted delivery are measured during various phases of treatment, including lung resistance and lung elastance.

European patent EP1292224B1 discloses an apparatus for displaying information obtained by electrical impedance tomography (EIT) data from a part of a patient's body. Impedance changes are interpreted as volume changes, regional volumes and flows are inferred from impedance variation over time, and regional compliance and resistance are derived from impedance-based regional spirometry. EP1292224B1 discloses that time dependent volumes can be determined on a regional as well as on a global basis. When observing volume changes versus time, a flow can be calculated and analysed for the entire lung, but also for a smaller region of interest within the lung. This way, pathologies with respect to compliance and resistance are detected not only on a global, but also on a regional lung level and the regional predominance of such a pathology can be identified. This European patent does not disclose any imaging of pleural motion, let alone lung sliding. It merely measures electrical properties of tissue, not mechanical displacement of the lung surface.

The following discloses certain improvements to overcome these problems and others.

## SUMMARY

A mechanical ventilation device includes an electronic controller configured to: receive imaging data and ventilator data associated with lungs of a patient while the patient undergoes mechanical ventilation therapy with an associated mechanical ventilator; determine a respiratory pressure of the patient based at least on the received ventilator data; determine a lung volume and a lung flow for at least two regions of the lungs based on lung sliding data determined from the received imaging data and the determined respiratory pressure; determine a regional resistance and elastance for the at least two regions of the lungs from the lung volume and the lung flow for the at least two regions; and display the regional resistance and elastance for the at least two regions of the lungs on a display device.

One advantage resides in providing regional lung information in a quick, accessible manner.

Another advantage resides in providing regional lung information without the use of CT imaging or EIT processes.

Another advantage resides in providing regional lung information for a patient who is providing an active respiratory effort.

Another advantage resides in providing regional lung information based on lung sliding measurements.

Another advantage resides in preventing VILI.

A given embodiment may provide none, one, two, more, or all of the foregoing advantages, and/or may provide other advantages as will become apparent to one of ordinary skill in the art upon reading and understanding the present disclosure.

## BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.

FIGURE 1 diagrammatically shows an illustrative mechanical ventilation system in accordance with the present disclosure.

FIGURES 2 and 3 diagrammatically illustrate typical anatomy of the lungs, with FIGURE 2 diagrammatically showing a front view including the parietal and visceral pleura and diagrammatically indicating illustrative regions L1, L2, R1, and R2, and FIGURE 3 diagrammatically showing side views of the left and right lungs and diagrammatically indicating illustrative regions L3-L6 and R3-R6.

FIGURE 4 shows an example flow chart of operations suitably performed by the system of FIGURE 1.

FIGURE 5 diagrammatically shows a lung sliding curve (bottom curves) for a constant driving pressure (top curve) of (Pao - Pmus).

## DETAILED DESCRIPTION

As used herein, the singular form of "a", "an", and "the" include plural references unless the context clearly dictates otherwise. As used herein, statements that two or more parts or components are "coupled," "connected," or "engaged" shall mean that the parts are joined, operate, or co-act together either directly or indirectly, i.e., through one or more intermediate parts or components, so long as a link occurs. Directional phrases used herein, such as, for example and without limitation, top, bottom, left, right, upper, lower, front, back, and derivatives thereof, relate to the orientation of the elements shown in the drawings and are not limiting upon the scope of the claimed invention unless expressly recited therein. The word "comprising" or "including" does not exclude the presence of elements or steps other than those described herein and/or listed in a claim. In a device comprised of several means, several of these means may be embodied by one and the same item of hardware.

Disclosed herein are systems and methods to support bedside clinicians and care providers in providing safe mechanical ventilation, including guiding clinicians in selecting ventilator settings that will not produce VILI in the specific patient.

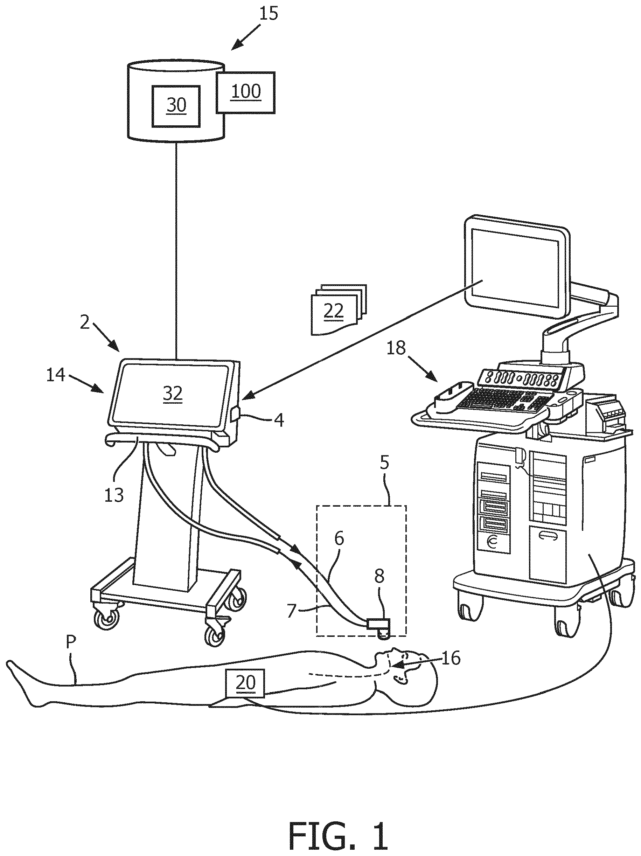

With reference to FIGURE 1, a mechanical ventilator **2** for providing ventilation therapy to an associated patient **P** is shown. As shown in FIGURE 1, the mechanical ventilator **2** includes an outlet **4** connectable with a patient breathing circuit **5** to delivery mechanical ventilation to the patient **P.** The patient breathing circuit **5** includes typical components for a mechanical ventilator, such as an inlet line **6,** an optional outlet line **7** (this may be omitted if the ventilator employs a single-limb patient circuit), a connector or port **8** for connecting with an endotracheal tube (ETT), and one or more breathing sensors (not shown), such as a gas flow meter, a pressure sensor, end-tidal carbon dioxide (etCO2) sensor, and/or so forth. The mechanical ventilator **2** is designed to deliver air, an air-oxygen mixture, or other breathable gas (supply not shown) to the outlet **4** at a programmed pressure and/or flow rate to ventilate the patient via an ETT. The mechanical ventilator **2** also includes an electronic controller (e.g., a microprocessor) **13** for controlling operation of the mechanical ventilator **2,** a display device **14** for displaying information about the patient **P** and/or settings of the mechanical ventilator **2** during mechanical ventilation of the patient **P,** and a non-transitory computer readable medium **15** storing instructions executable by the electronic controller **13.**

FIGURE 1 diagrammatically illustrates the patient **P** intubated with an ETT **16** (the lower portion of which is inside the patient **P** and hence is shown in phantom). The connector or port **8** connects with the ETT **16** to operatively connect the mechanical ventilator **2** to deliver breathable air to the patient **P** via the ETT **16.** The mechanical ventilation provided by the mechanical ventilator **2** via the ETT **16** may be therapeutic for a wide range of conditions, such as various types of pulmonary conditions like emphysema or pneumonia, viral or bacterial infections impacting respiration such as a COVID-19 infection or severe influenza, cardiovascular conditions in which the patient **P** receives breathable gas enriched with oxygen, or so forth.

FIGURE 1 also shows a medical imaging device **18** (also referred to as an image acquisition device, imaging device, and so forth). The illustrative image acquisition device **18** is an ultrasound (US) imaging device **18,** however in other embodiments it is contemplated for the image acquisition device to be a Computed Tomography (CT) image acquisition device, a C-arm imager, or other X-ray imaging device; Magnetic Resonance (MR) image acquisition device; or a medical imaging device of another modality. As primarily described herein, the medical imaging device **18** comprises a US imaging device with an ultrasound transducer **20** configured to acquire US images **22** of a diaphragm of the patient **P.** The US imaging device **18** can be a bedside imaging device. In some embodiments, the electronic controller **13** can be implemented in the US imaging device **18.**

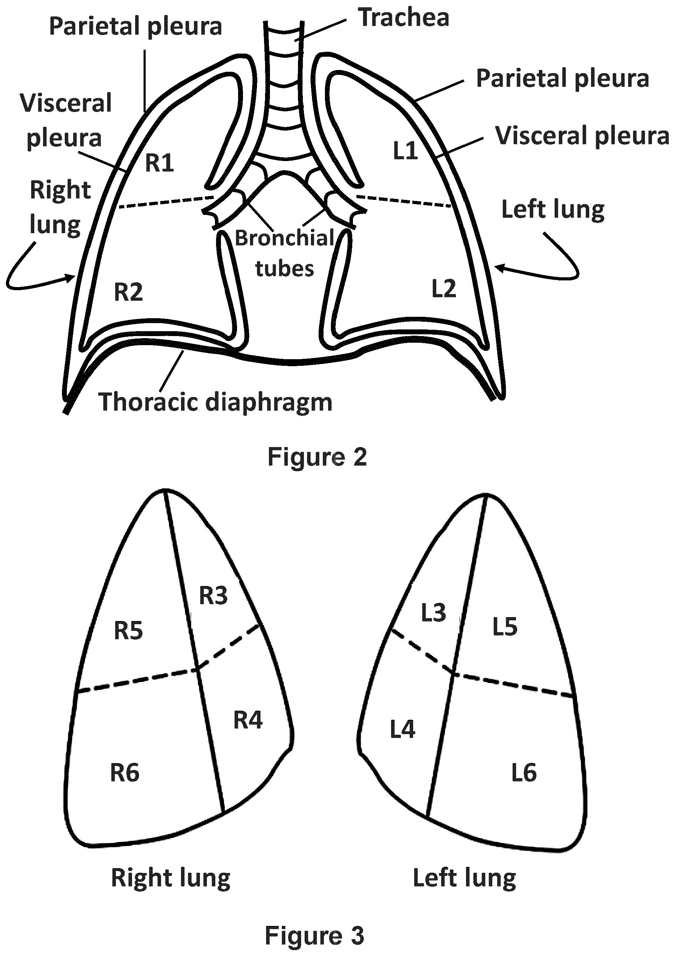

With brief reference to FIGURES 2 and 3, embodiments disclosed herein provide for assessing VILI risk factors on a regional basis. FIGURES 2 and 3 diagrammatically illustrate typical anatomy of the lungs, with FIGURE 2 diagrammatically showing a front view including the parietal and visceral pleura and diagrammatically indicating illustrative regions L1, L2, R1, and R2, and FIGURE 3 diagrammatically showing side views of the left and right lungs and diagrammatically indicating illustrative regions L3-L6 and R3-R6. These regions are merely illustrative, and other regions may be selected. For instance, the region may alternatively relate to five lung lobes. Moreover, it is contemplated for selected regions to spatially overlap. For example, the region R1 indicated in FIGURE 2 may be a combination of the regions R3 and R5 indicated in FIGURE 3.

With particular reference to FIGURE 2, the parietal and visceral pleura are also indicated, in the context of the left and right lungs, the trachea and bronchial tubes, and the thoracic diaphragm (also referred to herein for brevity as the diaphragm). The parietal and visceral pleura are tissue membranes, with the visceral pleura being disposed inside the parietal pleura. The visceral pleura is adherent to the outer surface of the lung, while the parietal pleura lines the thoracic wall. During respiration, relative movement between the visceral pleura and the parietal pleura is referred to as lung sliding.

As previously noted, such lung sliding manifests in ultrasound imaging as speckles, which are acoustic markers formed by interference of ultrasound waves that are scattered from physical structures of a size comparable to the wavelength of the ultrasound waves (such as the closely spaced parietal and visceral pleura). In some examples, a measure for lung sliding is suitably obtained from ultrasound imaging by calculating the average temporal intensity changes of the ultrasound speckles in the pleural line. This results in an Intensity Based Speckle Tracking (IBST) score waveform which correlates with the ventilator flow waveform. However, unlike the ventilator flow waveform which is a global parameter for the lungs as a whole, the IBST score waveform can be assessed for different regions of the lungs. For example, if one region of a lung is distending more than the rest of the lung during inspiration, this can be recognized as a different IBST score waveform for that region.

Referring back now to FIGURE 1, the non-transitory computer readable medium **15** of the mechanical ventilator **2** stores instructions executable by the electronic controller **13** to perform a mechanical ventilation method or process **100.** The mechanical ventilation method **100** can result in an output of regional resistance and compliance information of the lungs, and regional transpulmonary pressure information of the patient **P,** and/or a regional stress index based on assessment of the shape of the lung sliding-time curves. For example, the mechanical ventilation method **100** can be used to assess regional respiratory resistance and compliance/elastance using a regional lung model in combination with ultrasound-based diaphragm and lung sliding measurements. The model can be a simple lumped element model, a more advanced model of the specific lung region, or a pre-trained machine learning model. For example, the mechanical ventilation method **100** can solve various parameters of the mechanical ventilation therapy for multiple lung regions of the patient **P** according to Equation 1:

$$P_{ao}(t) - P_{mus}(t) = R_{regional}V_{regional}(t) + E_{regional}V_{regional}(t) + P0 \qquad (1)$$

wherein $P_{ao}(t)$ [cm H2O] is a pressure at an airway opening (i.e., the mouth), $P_{mus}(t)$ [cm H2O] is a pressure equivalent to an action of respiratory muscles, $R_{regional}$ [cm H2O *s/L] is a resistance of the respiratory system, $V_{regional}(t)$ [L/s] is a gas (air plus oxygen) flow through the airways, $E_{regional}$ ([cm H2O/L] is an elastance of the respiratory system (where a compliance $C_{regional}$ of the respiratory system is defined as $1/E_{regional}$), and $V_{regional}(t)$ (L) is a volume of inhaled gas and P0 is a constant term that can be estimated at any point at the end of the breath where the gas flow is zero and $P_{mus}(t)$ is zero (at which point P0 will be equal to Pao). The difference between $P_{ao}(t)$ and $P_{mus}(t)$ can be referred to as a driving pressure. The product of $R_{regional}V_{regional}(t)$ can be referred to as a resistive work. The product of $E_{regional}V_{regional}(t)$ can be referred to as elastic work. Equation 1 is a localized version of an equation of motion and assumes a driving pressure being the same for all regions of the lung.

In another example, to refine Equation 1, the elastance $E_{regional}$ is split into an elastance of the lung $E_{l,regional}$ and an elastance of the chest wall $E_{cw,regional}$. This allows the electronic controller **13** to solve for the transpulmonary pressure $P_{tp,regional}$ for multiple lung regions of the patient **P** according to Equations 2 to 5:

$$P_{ao}(t) - P_{alv,regional}(t) = R_{regional}V_{regional}(t) \qquad \textbf{Equation 2}$$

$$P_{tp,regional}(t) = P_{alv,regional}(t) - P_{pl,regional}(t) = \qquad \textbf{Equation 3}$$
$$E_{l,regional}V_{regional}(t)$$

$$P_{pl,regional}(t) - P_{mus}(t) = E_{cw,regional}V_{regional}(t) \qquad \textbf{Equation 4}$$

$$P_{tp,regional}(t) = E_{l,regional}V_{regional}(t) \qquad \textbf{Equation 5}$$

wherein $P_{alv,regional}(t)$ [cm H$_2$O] and $P_{pl,regional}(t)$ [cm H$_2$O] are the alveolar and pleural pressure of a specific lung region, respectively.

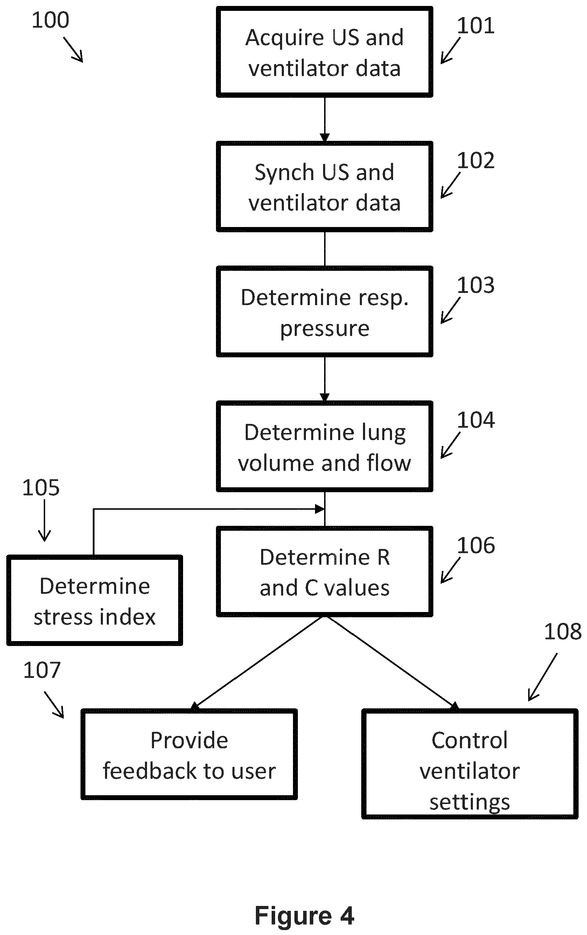

With reference to FIGURE 4, and with continuing reference to FIGURE 1, an illustrative embodiment of a mechanical ventilation method **100** is diagrammatically shown as a flowchart. To begin the method **100,** the patient **P** is intubated with the ETT **16** so that mechanical ventilation therapy with the mechanical ventilator **2** can begin.

At an operation **101,** imaging data (i.e., the US imaging data **22**) and ventilator (e.g., pressure or flow) data associated with lungs of the patient **P** are received at the mechanical ventilator **2**. The acquiring operation **101** can occur while the patient undergoes mechanical ventilation therapy with the associated mechanical ventilator **2**. The US imaging data **22** can comprise a mid-axillary intercostal approach at the zone of apposition, and/or a subcostal approach using the liver or spleen as an acoustic window (see, e.g., P. Tuinman et al. "Respiratory muscle ultrasonography: methodology, basic and advanced principles and clinical applications in ICU and ED patients-a narrative review", Intensive Care Med. 2020). The intercostal approach allows for easy real-time measurement of the diaphragm thickness and strain, whereas the diaphragm excursion can be more easily measured with the subcostal approach. In another example, surface electromyography (EMG) data can be obtained from the patient **P** by measuring auxiliary respiratory muscle activities of the patient **P.**

At an operation **102,** the received imaging data **22** and the received ventilator data are synchronized with respect to time. The mechanical ventilator **2** and the US imaging device **18** are two independent systems and often are from different manufacturers, which makes it a challenge to access clocks to synchronize them. Hence, these systems and the output data streams are often not synchronized. Also, additional synchronization processes may be needed since there still may be a delay between data measured by the mechanical ventilator **2** and the US imaging device **18.** Surface EMG data, if acquired, can also be synchronized in the same manner with the the received imaging data **22** and/or the received ventilator data.

Diaphragm thickness, strain, and excursion correlate well with ventilator waveforms generated by the mechanical ventilator **2** and hence can be used to synchronize the US imaging data **22** with ventilator waveforms (e.g., pressure or flow). For example, in pressure support ventilation the pressure ramps up at the onset of the thickening of the diaphragm. A time delay between the ventilator waveform and the US imaging data **22** can be determined, for example, by cross-correlation or by applying a pre-trained machine learning algorithm. This delay can be applied to other US imaging data **22,** for example, to measure lung sliding at various lung zones in order to synchronize these lung sliding measurements with the corresponding ventilator waveforms. The algorithm to determine the offset and to apply the offset to subsequent measurements can be run on the mechanical ventilator **2,** on the US imaging device **18,** on a separate processing unit or in the cloud.

Alternatively, diaphragm excursion could be used for synchronization. Also, a unique marker could be created for example with a gentle manual chest compression which results in specific features in the diaphragm thickness, strain and excursion and in the pressure and flow waveforms. In order to perform the synchronization procedure, the clinician could be asked to apply a chest compression within a certain time window (e.g., 10 s). This provides the time window in which the algorithm can search for the features to perform the synchronization. Feedback could be given to the caregiver in case of a successful synchronization or, in case the synchronization is not successful, the caregiver could be asked to repeat the chest compression. Other methods to provide unique markers could be through a dedicated breathing maneuver or the use of phrenic nerve stimulation to activate the diaphragm, e.g., with a unique pattern that could be searched for in the ultrasound and ventilator data by the algorithm. Also, one of the lung sliding measurements could be used to synchronize with the flow measured by the mechanical ventilator **2.**

At an operation **103,** a respiratory pressure of the lungs of the patient **P** is determined based on the received imaging data and the received ventilator data. This operation **103** corresponds to determining a pressure associated with the respiratory activity of the patient **P.** For example, the determined pressure corresponds to the driving pressure (i.e., $P_{ao}(t) - P_{mus}(t)$) of Equation 1. To determine the driving respiratory pressure, the airway opening pressure $P_{ao}(t)$ is determined from the ventilator pressure data, and the respiratory muscle pressure $P_{mus}(t)$ is determined from the US imaging data **22.** The driving respiratory pressure is determined from the difference between $P_{ao}(t)$ and $P_{mus}(t)$. Alternatively, the determined pressure corresponds to transpulmonary pressure of the patient **P.** In other examples, the determined pressure corresponds to any other pressure associated with respiratory of the patient, such as the respiratory muscle pressure $P_{mus}(t)$, a pressure at an opening of the esophagus of the patient **P** $P_{esophageal}(t)$, alveolar pressure $P_{alveolar}(t)$, a combination of any of these, and so forth.

In general, the diaphragm is the dominant respiratory muscle and hence the contribution of the accessory muscles to $P_{mus}$ can be neglected. For example, this can be done by the use of a force-length relation for muscle fibers where the length information is derived from the ultrasound measurements (see, e.g., Zhang et al. Biomechanical simulation of thorax deformation using finite element approach. BioMed Eng OnLine. 2016). Other methods can be employed to use ultrasound to assess and quantify the activity of the diaphragm. Diaphragm strain and strain rate correlate well with transdiaphragmatic pressure (see, e.g., E. Oppersma et al. Functional assessment of the diaphragm by speckle tracking ultrasound during inspiratory loading. J Appl Phys. 2017). The thickness fraction of the diaphragm corresponds well with the diaphragmatic pressure-time product (see, e.g., M. Umbrello et al. Diaphragm ultrasound as indicator of respiratory effort in critically ill patients undergoing assisted mechanical ventilation: a pilot clinical study. Crit Care. 2015). The thickness fraction (TFdi) is defined as the percentage inspiratory increase in the diaphragm thickness relative to end-expiratory thickness (Tee) during tidal breathing, as shown in Equation 2:

$$TFdi = \frac{Tei - Tee}{Tee} * 100\%$$

with Tei the end-inspiratory thickness. In some examples, the surface EMG data can assist in assessing the respiratory muscle pressure by measuring auxiliary respiratory muscle activities as an additional input. Surface EMG data synchronized with the imaging data **22** and/or the ventilator data can improve the accuracy of the respiratory muscle activities estimation.

It is also noted that in the case of a passive patient who is not exerting any respiratory effort (for example, a fully sedated patient), the respiratory muscle pressure $P_{mus}(t)$ is equal to zero.

At an operation **104,** a lung volume (i.e., the volume of inhaled gas $V_{regional}(t)$) and a lung flow (i.e., gas flow into a region of the lung $V_{regional}(t)$) for at least one region of the lungs of the patient **P** can be determined based on lung sliding data and the determined respiratory pressure of the lungs (i.e., the driving pressure). To determine the lung sliding data, additional imaging data **22** of each region of the lungs of the patient **P** is obtained and sliding waveforms for each region of the lungs of the patient **P** are generated from the additional imaging data **22.** This operation **104** is repeated for each regional of the lungs of the patient **P.**

Lung sliding US measurements are obtained with the US imaging device **18** at various lung zones. The lung sliding is quantified and the lung sliding waveforms (i.e., one waveform per region of the lungs) are synchronized with the ventilator waveforms using the time delay determined at the operation **102.**

Regional lung sliding depends on several parameters such as the regional lung resistance and compliance but also on global parameters such as the shape and size of the chest wall and the diaphragm. Also, it is important to know at which location the lung sliding is measured. For example, since the diaphragm expands the lung in downward direction while the lung is 'fixated' at the upper end the amount of lung sliding in the lower lung zones is expected to be larger than in the upper zones. To address this, the volume $V_{regional}(t)$ and the flow $V_{regional}(t)$ can be determined at each region of the lungs using a (biomechanical) model **30** of the lungs of the patient **P** with imaging data of dimensions of the lungs and diaphragm of the patient **P.** For example, the model **30** of the patient **P** can be generated from previously acquired imaging data (i.e., CT imaging data) and stored in the non-transitory computer readable medium **15** of the mechanical ventilator **2.** The model **30** can be retrieved, and then used to determine the volume $V_{regional}(t)$ and the flow $V_{regional}(t)$ based on the imaging data of dimensions of the lungs and diaphragm of the patient **P.**

To construct the model **30,** a machine learning model can be trained for each lung region using a data set comprising simultaneously recorded lung sliding data and CT images. Local volume changes can be derived from the CT images and serve as ground truth values for the training. For each region, local volume changes can be inferred by applying the trained machine learning model to patient specific lung sliding data from that specific region. Optionally, prior to use, the trained machine learning model is calibrated with patient specific CT images.

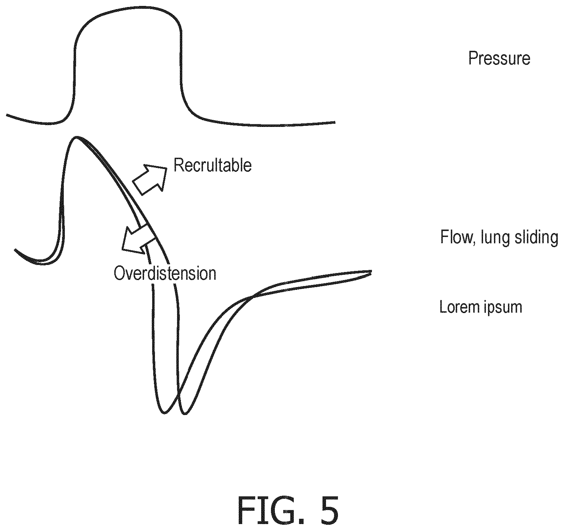

With continuing reference to FIGURE 4 and further reference to FIGURE 5, in some embodiments, at an optional operation **105,** a regional stress index based on assessment of the shape of the lung sliding-time curves can be generated. For a period of constant driving pressure (i.e., $P_{ao}(t) - P_{mus}(t)$), the air flow should show a linear decay, like the shape of the pressure-time curve for constant flow. US lung sliding can be used as a measure for regional flow. FIGURE 5 diagrammatically shows a lung sliding curve (bottom curves) for a constant driving pressure (top curve). As there seen, if the shape of the lung sliding-time curve is deviating from the linear trajectory, it indicates the presence of tidal recruitment or overdistension. The constant driving pressure can be assured by measuring $P_{ao}(t)$ from the mechanical ventilator **2** and $P_{mus}(t)$ from diaphragmatic US imaging data **22.** For each lung zone a local stress index can be determined indicating tidal recruitment or overdistension.

The generated sliding waveforms of each region of the lungs of the patient **P** can be used to determine a stress index for the at least one region of the lungs based on the lung sliding data and the driving respiratory pressure. To do so, the generated sliding waveforms of each region of the lungs of the patient **P** includes a regional stress index for each region of the lungs. In some examples, the mechanical ventilator **2** can be controlled to adjust one or more parameters of the mechanical ventilation therapy delivered to the patient **P** based on the generated lung sliding-time curves having the regional stress index. The generated lung sliding-time curves can be displayed on the display device **14** of the mechanical ventilator **2.**

In case the driving pressure is not constant during inhalation (e.g., a non-zero $P_{mus}(t)$ or a time-variable $P_{ao}(t)$), several methods are proposed to indicate presence of (de)recruitment at exhalation and/or overdistension at inhalation. In one example, the driving pressure can be constructed from the measured ultrasound $P_{mus}(t)$ and ventilator $P_{ao}(t)$ waveforms and the expected flow behaviour can be predicted based on a single compartment model with constant resistance and compliance values. Then, the expected lung sliding can be predicted from the biomechanical model **30** of the respiratory system and the predicted lung sliding behaviour can be compared with the measured lung sliding. A deviation between the measured and predicted lung sliding behaviour indicates presence of a volume-dependent compliance (assuming volume dependency of resistance to be small) which, depending on the sign and shape of the error curve, indicates presence of (de)recruitment or overdistension. In another example, a single compartment model can be fit with constant resistance and compliance values to the measured waveforms and split the fit residual in several portions throughout the breathing cycle. Depending on whether the fit residual increases or decreases presence of (de) recruitment or overdistension can be derived. In another example, the breathing cycle can be split in several portions to construct the biomechanical model **30** with different compliance values for each portion and fit this to the measured waveforms. The behaviour of the fitted time-variable compliance (different compliance values for each compartment) indicates presence of decruitment (increasing compliance) or overdistension (decreasing compliance).

In some examples, a presence of regional (de)recruitment or overdistension can be assessed from a regional pressure-volume loop. To this end, the regional volume is derived from lung sliding measurements and the biomechanical model **30**. To construct the regional pressure-volume loop, a global transpulmonary pressure waveform is plotted against the regional volume waveform for one or more breathing cycles. The global transpulmonary pressure can be determined using known methods such as the use of esophageal pressure. Another method to construct the regional pressure-volume loop is by plotting a regional transpulmonary pressure waveform against the regional volume waveform for one or more breathing cycles. The regional transpulmonary pressure can be determined from the operation **106**. Alternatively, information on regional (de)recruitment or overdistension is derived directly from a pressure-lung sliding loop. The information from the various regional pressure-volume loops is used to guide ventilation, e.g., PEEP and tidal volume level such that the majority of the lung regions are ventilated in the safe window or such that no or as little as possible lung regions show severe decruitment or overdistension.

Additionally, the regional pressure-volume (or -lung sliding) loop can be combined with additional information extracted from the US Imaging data **22** such as B-lines to identify a presence of atelectasis. For example, for several PEEP values, a regional pressure-volume loop could be constructed and for each PEEP setting the number of B-lines could be identified such as to provide information on optimum PEEP values. Similarly, these measurements could be combined with dynamic air bronchograms to identify decruitment.

At an operation **106**, a regional resistance (i.e., the resistance $R_{regional}$ of a region of a lung) and a regional elastance (i.e., the elastance $E_{regional}$ of the region of the lung) for the at least one region of the lungs of the patient **P** is determined from the lung volume $V_{regional}(t)$ and the lung flow $V_{regional}(t)$. To do so, the elastance $E_{regional}$ is first determined when the lung flow $V_{regional}(t)$ is zero (i.e., the resistive work is Equation 1 is zero). The elastance $E_{regional}$ can then be determined by the driving respiratory pressure $P_{ao}(t) - P_{mus}(t)$ (from the operation **103**) and the lung volume $V_{regional}(t)$ (from the operation **104**) by solving Equation 1. Once the elastance $E_{regional}$ is known, Equation 1 can then be solved for the resistance $R_{regional}$ based on the (non-zero) lung flow $V_{regional}(t)$ determined from the operation **104**.

In some embodiments, at the operation **106**, a regional transpulmonary pressure for the at least one lung region is determined. To do so, an elastance of the chest wall $E_{cw,regional}$ of a selected lung region is first determined, for example using an algorithm to compute the chest wall elastance based on a computed tomography (CT) scan. A finite element model of the region of the thorax can be constructed based on a segmentation of the CT scan (see, e.g., Zhang et al. BioMed Eng OnLine (2016) 15:18, "Biomechanical simulation of thorax deformation using finite element approach"). Known mechanical properties of the structures (intercostal muscle, diaphragm, bone, cartilage, tendons, and so forth) are input to the finite element model. The effective chest wall elastance of that region can be calculated by imposing a force F (or a pressure) in a direction perpendicular to the chest wall (as a boundary condition), and subsequently determine the simulated displacement x from the model output: $E_{cw,regional} = F/x$. Alternatively, the chest wall compliance can be assumed from values from a lookup table that uses known values.

Next, from Equation 4 the pleural pressure $P_{pl,regional}(t)$ of the selected lung region is calculated from the chest wall elastance $E_{cw,regional}$, the lung volume $V_{regional}(t)$ and respiratory muscle pressure $P_{mus}(t)$. Then, from Equation 2 the alveolar pressure $P_{alv,regional}(t)$ of the selected lung region is calculated from the resistance $R_{regional}$, lung flow $V_{regional}(t)$ and airway opening pressure $P_{ao}(t)$. Next, from $E_{regional} = E_{l,regional} + E_{cw,regional}$ the lung elastance $E_{l,regional}$ for the selected lung region is calculated. Finally, from Equation 3 the regional transpulmonary pressure $P_{tp,regional}(t)$ is calculated from the pleural pressure $P_{pl,regional}(t)$, alveolar pressure $P_{alv,regional}(t)$, lung elastance $E_{l,regional}$ and lung volume $V_{regional}(t)$.

The operation **106** can be performed in cases where $P_{mus}$ is zero or non-zero. Diaphragmatic ultrasound can be used to verify that $P_{mus}$ is zero, for example, by measuring the thickness fraction of the diaphragm (TFdi) at the intercostal space which should be zero or slightly negative due to passive stretching (see, e.g., Santana PV, Cardenas LZ, Albuquerque ALP, Carvalho CRR, Caruso P. Diaphragmatic ultrasound: a review of its methodological aspects and clinical uses. J Bras Pneumol. 2020 Nov 20), or by constructing $P_{mus}$ from a force-length relation for muscle fibers. There are several ways to estimate the regional compliance $C_{regional} = 1/E_{regional}$. For example, the pressure at the airway $P_{ao}$ is measured by the ventilator at the end of inhalation and exhalation where the gas flow $V_{regional}$ is zero such that the resistive work in Equation 1 is zero. Simultaneously, the regional volume $V_{regional}(t)$ is derived from ultrasound-based lung sliding measurements (from the operation **105**). Once the regional compliance is obtained, regional resistance can be estimated by applying Equation 1 on a part of the breathing cycle with non-zero gas flow $V_{regional}$. Also, other methods such as a least squares regression can be used.

A second case concerns an actively breathing patient where $P_{mus}$ is nonzero. The approach to determine regional resistance and compliance is similar to the approach for the passive patient, where instead of $P_{mus}$ equals zero, the $P_{mus}$ waveform from the operation **102** is used as input to Equation 1. A similar approach could be used for each region of the lungs.

At an operation **107,** the regional resistance $R_{regional}$ and elastance $E_{regional}$ for the at least one region of the lungs of the patient **P** can be displayed on the display device **14.** In some embodiments, a graph **32** showing changes of the regional resistance and elastance for the at least one region of the lungs as a function of time can be displayed on the display device **14.** In some examples, a user input can be provided to a portion of the displayed graph **32** (i.e., a user or clinician providing a finger tap to a portion of the graph **32**), and additional information related to the portion of the graph **32** can be displayed where the user input was received (i.e., a clinician can provide a finger tap at, for example, a time of 5 seconds into the mechanical ventilation therapy to determine the resistance and elastance at the 5 second time mark).

The displayed graph **32** can be used to give feedback to the clinician. For example, a regional intrinsic PEEP (iPEEP) value can be detected from the lung sliding measurements. To this end, the regional lung sliding measurements are converted into regional flow measurements. Next, for each lung region the flow at end of exhalation is determined. If the end-of-exhalation flow is not zero, the ventilation settings are adapted such as to provide a longer exhalation time and again the regional end-of-exhalation flow is assessed. This is repeated until the end-of- exhalation flow is zero or does not decrease further.

For example, the effect of ventilation parameters such as PEEP levels on the presence of atelectasis/tidal recruitment and overdistension for the various lung zones to guide the ventilation therapy is visualized. Also, regional resistance and compliance values can be presented as function of time to see temporal changes in regional lung status. For example, in case the regional resistance is changing it could be suggested to provide a bronchodilator or to apply suctioning to remove mucus.

In another example, pendelluft can be detected from regional lung sliding. To this end, the US based lung sliding measurements need to be synchronized as in the operation 102. The regional lung sliding measurements are converted into regional flow measurements. From the various flow patterns the presence of pendelluft is detected. Alternatively, presence of pendelluft is detected directly from the (synchronized) lung sliding measurements.

At an operation **108,** the mechanical ventilator **2** can be controlled to adjust one or more parameters of the mechanical ventilation therapy delivered to the patient **P** using the regional resistance $R_{regional}$ and elastance $E_{regional}$.

The disclosure has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims .

**Claims**

1. A mechanical ventilation device, comprising an electronic controller configured to:

   receive imaging data and ventilator data associated with lungs of a patient while the patient undergoes mechanical ventilation therapy with an associated mechanical ventilator;
   determine a respiratory pressure (103) of the patient based at least on the received ventilator data;

   wherein the electronic controller is further configured to:

   determine a lung volume and a lung flow (104) for at least two regions of the lungs based on lung sliding data determined from the received imaging data and the determined respiratory pressure;
   determine a regional resistance and elastance (106) for the at least two regions of the lungs from the lung volume and the lung flow for the at least two regions; and
   display the regional resistance and elastance for the at least two regions of the lungs on a display device.

2. The device of claim 1, wherein the electronic controller is further configured to determine a respiratory pressure of the patient based on the received ventilator data and further based on the received imaging data by:

   determining an airway opening pressure from the ventilator data; determining a respiratory muscle pressure from the imaging data; and
   determining the respiratory pressure of the patient from the airway opening pressure and the respiratory muscle pressure.

3. The device of claim 1, wherein the electronic controller is configured to:
   synchronize the received imaging data and the received ventilator data with respect to time.

4. The device of claim 1, wherein the electronic controller is configured to determine the lung sliding data based on tracking of speckles in the received imaging data.

5. The device of claim 1, wherein the electronic controller is configured to determine the regional elastance for the at least two regions of the lungs by:
calculating the regional elastance for the at least two regions of the lungs using the received imaging data, the received ventilator data, and the lung volume when the lung flow for the at least two regions of the lungs is zero.

6. The device of claim 5, wherein the electronic controller is configured to determine the regional resistance for the at least two regions of the lungs by:
calculating the regional resistance for the at least two regions of the lungs using the received imaging data, the received ventilator data, the lung flow for the at least two regions of the lungs, and the calculated regional elastance for the at least two regions of the lungs.

7. The device of claim 5, wherein the electronic controller is configured to:
determine a regional transpulmonary pressure for the at least two regions of the lungs from the determined regional elastance and the determined lung volume of the at least two regions of the lungs.

8. The device of claim 7, wherein the electronic controller is configured to:
control the associated mechanical ventilator to adjust one or more parameters of the mechanical ventilation therapy delivered to the patient based on the regional transpulmonary pressure for the at least two regions of the lungs.

9. The device of claim 1, wherein the at least one electronic controller is configured to determine the lung volume and a lung flow for the at least two regions of the lungs by:
generating a model of the lungs with imaging data of dimensions of the lungs and diaphragm of the patient.

10. The device of claim 1, wherein the electronic controller is configured to:
display, on the display device, a graph showing changes of the regional resistance and elastance for the at least two regions of the lungs as a function of time.

11. The device of claim 1, wherein the electronic controller is configured to:
determine a stress index (105) for the at least two regions of the lungs based on the lung sliding data, the received imaging data, and the received ventilator data.

12. The device of claim 11, wherein the electronic controller is configured to determine the stress index by:

obtaining additional imaging data of the at least two regions of the lungs of the patient;
generating sliding waveforms for the at least two regions of the lungs of the patient; and
generating lung sliding-time curves with regional stress index for the at least two regions of the lungs.

13. The device of claim 11, wherein the electronic controller is configured to:
determine at least one setting of the mechanical ventilation therapy based on the generated lung sliding-time curves having the regional stress index for the at least two regions of the lungs.

14. The device of claim 1, wherein the electronic controller is configured to:
control the associated mechanical ventilator to adjust one or more parameters of the mechanical ventilation therapy delivered to the patient based on the determined regional resistance and elastance for the at least two regions of the lungs.

**Patentansprüche**

1. Mechanische Beatmungsvorrichtung, umfassend eine elektronische Steuereinheit, die für Folgendes ausgelegt ist:

Empfangen von Bildgebungsdaten und Beatmungsgerätedaten der Lunge eines Patienten während der Beatmungstherapie des Patienten mit einem zugehörigen Beatmungsgerät;
Ermitteln eines Atemdrucks (103) des Patienten mindestens auf der Grundlage der empfangenen Beatmungsgerätedaten;
wobei die elektronische Steuereinheit ferner für Folgendes ausgelegt ist:

Ermitteln eines Lungenvolumen und eines Lungenflows (104) für mindestens zwei Lungenregionen auf der Grundlage von Daten zum Lungengleiten, die aus den empfangenen Bildgebungsdaten und dem ermittelten Atemdruck ermittelt werden;

Ermitteln eines regionalen Widerstands und Elastizität (106) für die mindestens zwei Lungenregionen aus dem Lungenvolumen und dem Lungenflow für die mindestens zwei Regionen; und

Anzeigen des regionalen Widerstands und Elastizität für die mindestens zwei Lungenregionen auf einer Anzeigevorrichtung.

2. Vorrichtung nach Anspruch 1, wobei die elektronische Steuereinheit ferner so ausgelegt ist, dass sie den Atemdruck des Patienten auf der Grundlage der empfangenen Beatmungsgerätedaten und ferner auf der Grundlage der empfangenen Bildgebungsdaten ermittelt durch:

Ermitteln eines Atemwegsöffnungsdrucks aus den Beatmungsgerätedaten;

Ermitteln eines Atemmuskeldrucks aus den Bildgebungsdaten; und

Ermitteln des Atemdrucks des Patienten aus dem Atemwegsöffnungsdruck und dem Atemmuskeldruck.

3. Vorrichtung nach Anspruch 1, wobei die elektronische Steuereinheit für Folgendes ausgelegt ist:
zeitliches Synchronisieren der empfangenen Bildgebungsdaten und der empfangenen Beatmungsgerätedaten.

4. Vorrichtung nach Anspruch 1, wobei die elektronische Steuereinheit so ausgelegt ist, dass sie die Daten zum Lungengleiten auf der Grundlage der Verfolgung von Speckles in den empfangenen Bildgebungsdaten ermittelt.

5. Vorrichtung nach Anspruch 1, wobei die elektronische Steuereinheit so ausgelegt ist, dass sie die regionale Elastizität für die mindestens zwei Lungenregionen folgendermaßen ermittelt:
Berechnen der regionalen Elastizität für die mindestens zwei Lungenregionen unter Verwendung der empfangenen Bildgebungsdaten, der empfangenen Beatmungsgerätedaten und des Lungenvolumens, wenn der Lungenflow für die mindestens zwei Lungenregionen null beträgt.

6. Vorrichtung nach Anspruch 5, wobei die elektronische Steuereinheit so ausgelegt ist, dass sie den regionalen Widerstand für die mindestens zwei Lungenregionen folgendermaßen ermittelt durch:
Berechnen des regionalen Widerstands für die mindestens zwei Lungenregionen unter Verwendung der empfangenen Bildgebungsdaten, der empfangenen Beatmungsgerätedaten, des Lungenflows für die mindestens zwei Lungenregionen und der berechneten regionalen Elastizität für die mindestens zwei Lungenregionen.

7. Vorrichtung nach Anspruch 5, wobei die elektronische Steuereinheit für Folgendes ausgelegt ist:
Ermitteln eines regionalen transpulmonalen Drucks für die mindestens zwei Lungenregionen aus der ermittelten regionalen Elastizität und dem ermittelten Lungenvolumen der mindestens zwei Lungenregionen.

8. Vorrichtung nach Anspruch 7, wobei die elektronische Steuereinheit für Folgendes ausgelegt ist:
Steuern des zugehörigen Beatmungsgeräts, um einen oder mehrere Parameter der Beatmungstherapie für den Patienten auf der Grundlage des regionalen transpulmonalen Drucks für die mindestens zwei Lungenregionen anzupassen.

9. Vorrichtung nach Anspruch 1, wobei die mindestens eine elektronische Steuereinheit so ausgelegt ist, dass sie das Lungenvolumen und einen Lungenflow für die mindestens zwei Lungenregionen durch Folgendes ermittelt:
Erstellen eines Lungenmodells mit Bildgebungsdaten der Abmessungen der Lunge und des Zwerchfells des Patienten.

10. Vorrichtung nach Anspruch 1, wobei die elektronische Steuereinheit für Folgendes ausgelegt ist:
Anzeigen, auf der Anzeigevorrichtung, eines Diagramms, das Veränderungen des regionalen Widerstands und Elastizität für die mindestens zwei Lungenregionen in Abhängigkeit von der Zeit zeigt.

11. Vorrichtung nach Anspruch 1, wobei die elektronische Steuereinheit für Folgendes ausgelegt ist: Ermitteln eines Belastungsindex (105) für die mindestens zwei Lungenregionen auf der Grundlage der Daten zum Lungengleiten, der empfangenen Bildgebungsdaten und der empfangenen Beatmungsgerätedaten.

12. Vorrichtung nach Anspruch 11, wobei die elektronische Steuereinheit so ausgelegt ist, dass sie den Belastungsindex folgendermaßen ermittelt:

Gewinnen zusätzlicher Bildgebungsdaten von den mindestens zwei Lungenregionen des Patienten;
Erzeugen von Wellenformen zum Gleiten für die mindestens zwei Lungenregionen des Patienten; und
Erzeugen von Zeitkurven zum Lungengleiten mit regionalem Belastungsindex für die mindestens zwei Lungenregionen.

13. Vorrichtung nach Anspruch 11, wobei die elektronische Steuereinheit für Folgendes ausgelegt ist: Ermitteln mindestens einer Einstellung der Beatmungstherapie auf der Grundlage der erzeugten Zeitkurven zum Lungengleiten mit dem regionalen Belastungsindex für die mindestens zwei Lungenregionen.

14. Vorrichtung nach Anspruch 1, wobei die elektronische Steuereinheit für Folgendes ausgelegt ist: Steuern des zugehörigen Beatmungsgeräts, um einen oder mehrere Parameter der Beatmungstherapie des Patienten auf der Grundlage des ermittelten regionalen Widerstands und Elastizität für die mindestens zwei Lungenregionen anzupassen.

**Revendications**

1. Dispositif de ventilation mécanique, comprenant un dispositif de commande électronique configuré pour :

recevoir des données d'imagerie et des données de ventilateur associées aux poumons d'un patient pendant que le patient subit une thérapie de ventilation mécanique à l'aide d'un ventilateur mécanique associé ;
déterminer une pression respiratoire (103) du patient sur la base au moins des données de ventilateur reçues ;
dans lequel le dispositif de commande électronique est en outre configuré pour :

déterminer un volume pulmonaire et un flux pulmonaire (104) pour au moins deux régions des poumons sur la base de données de glissement pulmonaire déterminées à partir des données d'imagerie reçues et de la pression respiratoire déterminée ;
déterminer une résistance et une élastance régionales (106) pour les au moins deux régions des poumons à partir du volume pulmonaire et du flux pulmonaire pour les au moins deux régions ; et
afficher sur un dispositif d'affichage la résistance et l'élastance régionales des au moins deux régions des poumons.

2. Dispositif selon la revendication 1, dans lequel le dispositif de commande électronique est en outre configuré pour déterminer une pression respiratoire du patient à partir des données du ventilateur reçues et en outre sur la base des données d'imagerie reçues par l'intermédiaire des étapes consistant à :

déterminer une pression d'ouverture des voies aériennes à partir des données de ventilateur ;
déterminer une pression des muscles respiratoires à partir des
données d'imagerie ; et
déterminer la pression respiratoire du patient à partir de la pression d'ouverture des voies aériennes et de la pression des muscles respiratoires.

3. Dispositif selon la revendication 1, dans lequel le dispositif de commande électronique est configuré pour :
synchroniser les données d'imagerie reçues et les données de ventilateur reçues par rapport au temps.

4. Dispositif selon la revendication 1, dans lequel le dispositif de commande électronique est configuré pour déterminer les données de glissement pulmonaire sur la base du suivi de taches dans les données d'imagerie reçues.

5. Dispositif selon la revendication 1, dans lequel le dispositif de commande électronique est configuré pour déterminer l'élastance régionale des au moins deux régions des poumons par l'intermédiaire du :
calcul de l'élastance régionale pour les au moins deux régions des poumons à l'aide des données d'imagerie reçues, des données de ventilateur reçues et du volume pulmonaire lorsque le flux pulmonaire pour les au moins deux régions des poumons est nul.

6. Dispositif selon la revendication 5, dans lequel le dispositif de commande électronique est configuré pour déterminer la résistance régionale pour les au moins deux régions des poumons par l'intermédiaire du :
calcul de la résistance régionale pour les au moins deux régions des poumons en utilisant les données d'imagerie reçues, les données de ventilateur reçues, le flux pulmonaire pour les au moins deux régions des poumons, et l'élastance régionale calculée pour les au moins deux régions des poumons.

7. Dispositif selon la revendication 5, dans lequel le dispositif de commande est en outre configuré pour :
déterminer une pression transpulmonaire régionale pour les au moins deux régions des poumons à partir de l'élastance régionale déterminée et du volume pulmonaire déterminé des au moins deux régions des poumons.

8. Dispositif selon la revendication 7, dans lequel le dispositif de commande électronique est configuré pour :
commander le ventilateur mécanique associé pour ajuster un ou plusieurs paramètres de la thérapie de ventilation mécanique administrée au patient sur la base de la pression transpulmonaire régionale pour les au moins deux régions des poumons.

9. Dispositif selon la revendication 1, dans lequel le au moins un dispositif de commande électronique est configuré pour déterminer le volume pulmonaire et un flux pulmonaire pour les au moins deux régions des poumons par l'intermédiaire de :
la génération d'un modèle des poumons avec des données d'imagerie de dimensions des poumons et du diaphragme du patient.

10. Dispositif selon la revendication 1, dans lequel le dispositif de commande électronique est configuré pour :
afficher, sur le dispositif d'affichage, un graphique montrant des variations de la résistance et de l'élastance régionales pour les au moins deux régions des poumons en fonction du temps.

11. Dispositif selon la revendication 1, dans lequel le dispositif de commande électronique est configuré pour : déterminer un indice de contrainte (105) pour les au moins deux régions des poumons sur la base des données de glissement pulmonaire, des données d'imagerie reçues et des données de ventilateur reçues.

12. Dispositif selon la revendication 11, dans lequel le dispositif de commande électronique est configuré pour déterminer l'indice de contrainte par l'intermédiaire de :

l'obtention de données d'imagerie supplémentaires des au moins deux régions des poumons du patient ;
la génération de formes d'onde glissantes pour les au moins deux régions des poumons du patient ; et
la génération de courbes de glissement pulmonaire-temps avec un indice de contrainte régional pour les au moins deux régions des poumons.

13. Dispositif selon la revendication 11, dans lequel le dispositif de commande électronique est configuré pour :
déterminer au moins un réglage de la thérapie de ventilation mécanique sur la base des courbes de glissement pulmonaire-temps générées présentant l'indice de contrainte régional pour les au moins deux régions des poumons.

14. Dispositif selon la revendication 1, dans lequel le dispositif de commande électronique est configuré pour :
commander le ventilateur mécanique associé afin d'ajuster un ou plusieurs paramètres de la thérapie de ventilation mécanique délivrée au patient sur la base de la résistance et de l'élastance régionales déterminées pour les au moins deux régions des poumons.

# FIG. 1

Figure 2

Figure 3

100

101
Acquire US and
ventilator data

102
Synch US and
ventilator data

103
Determine resp.
pressure

104
Determine lung
volume and flow

105

106
Determine R
and C values

Determine
stress index

107

108

Provide
feedback to user

Control
ventilator
settings

**Figure 4**

Pressure

Recruitable

Flow, lung sliding

Overdistension

Lorem ipsum

FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63426069
- US 2015290418 A1

- EP 1292224 B1

**Non-patent literature cited in the description**

- **LICHTENSTEIN DA et al.** Relevance of lung ultrasound in the diagnosis of acute respiratory failure: the BLUE protocol. *Chest*, 2008
- **LICHTENSTEIN, D.A**. Lung ultrasound in the critically ill. *Ann. Intensive Care*, 2014, vol. 4
- **TUSMAN G et al.** Real-time images of tidal recruitment using lung ultrasound. *Crit Ultrasound J.*, 2015
- **G. DUCLOS et al.** Speckle tracking quantification of lung sliding for the diagnosis of pneumothorax: A multicentric observational study. *Intensive Care Med.*, 2019
- **G. DUCLOS et al.** A picture's worth a thousand words: Speckle tracking for quantification and assessment of lung sliding. *Intensive care med*, 2019
- **E. FISSORE et al.** Pneumothorax diagnosis with lung sliding quantification by speckle tracking: A prospective multicentric observational study. *The American journal of emergency medicine*, 2021
- **L. CROGNIER et al.** Diaphragmatic speckle tracking imaging for 2D-strain assessment in mechanical ventilation weaning test. *Medical hypotheses*, 2021

- **P. TUINMAN et al.** Respiratory muscle ultrasonography: methodology, basic and advanced principles and clinical applications in ICU and ED patients-a narrative review. *Intensive Care Med.*, 2020
- **ZHANG et al.** Biomechanical simulation of thorax deformation using finite element approach. *BioMed Eng OnLine*, 2016
- **E. OPPERSMA et al.** Functional assessment of the diaphragm by speckle tracking ultrasound during inspiratory loading. *J Appl Phys.*, 2017
- **M. UMBRELLO et al.** Diaphragm ultrasound as indicator of respiratory effort in critically ill patients undergoing assisted mechanical ventilation: a pilot clinical study.. *Crit Care*, 2015
- **ZHANG et al.** Biomechanical simulation of thorax deformation using finite element approach. *BioMed Eng OnLine*, 2016, vol. 15, 18
- **SANTANA PV** ; **CARDENAS LZ** ; **ALBUQUERQUE ALP** ; **CARVALHO CRR** ; **CARUSO P**. Diaphragmatic ultrasound: a review of its methodological aspects and clinical uses.. *J Bras Pneumol.*, 20 November 2020